# EUROPÄISCHE PATENTSCHRIFT

(11) **EP 0 393 081 B1**
(45) Veröffentlichungstag und Bekanntmachung des Hinweises auf die Patenterteilung: **10.02.1993**
(21) Anmeldenummer: 88909744.0
(22) Anmeldetag: 11.11.1988
(51) Int. Cl.: A61F 5/01

(54) **ORTHESE FÜR DAS MENSCHLICHE KNIE**
ORTHESIS FOR THE HUMAN KNEE
ORTHESE DU GENOU HUMAIN

(30) Priorität: 13.11.1987 DE 3738664
(43) Veröffentlichungstag der Anmeldung: 24.10.1990
(73) Patentinhaber: Bauerfeind GmbH & Co., D-47880 Kempen (DE)
(72) Erfinder: KUEHNEGGER, Walter, Tucson, AZ 85715 (US); BAUERFEIND, Hans, B., W-4152 Kempen 1 (DE)
(74) Vertreter: Bardehle, Heinz, Dipl.-Ing.
(86) Internationale Anmeldenummer: DE8800703
(87) Internationale Veröffentlichungsnummer: WO8904155

(56) Entgegenhaltungen:
- EP-A- 0 070 411
- GB-A- 2 177 603
- Prospekt der Firma "Orthopädie-Technik GmbH BIEDERMANN"
- Medizinische-Orthopädische Technik, Band 101, Nr. 2, März /April 1981, Gentner Verlag, (Stuttgart, DE), A. Bähler: "Die Othopädietechnische Versorgung des Knies beim Sportler",siehe Seiten 32-37
- Medizinisch-Orthopädische Technik, Band 104, Nr. 2, März /April 1984, Gentner Verlag, (Stuttgart, DE),E. Marquardt et al.:"Konservative Therapie bei Arthrosen - Möglichkeiten der orthetischen Versorgung",siehe Seite 39-46
- Medizinisch-Orthopädische Technik, Band 107, Nr. März/April 1987, Gentner Verlag, (Stuttgart, DE), W. Knoche: "Knieorthesen - Schwerpunkte der postoperativen Kniebehandlung", siehe Seiten 64-67, insbesondere Seite 65, Teil 2

## Beschreibung

Die Erfindung bezieht sich auf eine Orthese für das menschliche Knie mit zwei an den Seiten des Knies angeordneten Gelenken, aus denen die Kniescheibe umfassende Arme herausragen, die paarweise über und unter der Kniescheibe jeweils in einer mittig angeordneten Lasche zusammenlaufen, von der oberhalb und unterhalb des Knies mindestens je eine Oberschenkel- und Unterschenkel-Schelle getragen werden.

Eine derartige Orthese ist bekannt (siehe Prospekt der Firma "Orthopädie-Technik GmbH BIEDERMANN"). Zur Anpassung an das Bein des jeweiligen Patienten wird von dem Bein eine Umrißzeichnung angefertigt und nach dieser Zeichnung oder einem entsprechenden Gipsmodell dann die Orthese individuell erstellt.

Der Erfindung liegt die Aufgabe zugrunde, eine Orthese zu schaffen, die zur Sofort-Versorgung eines Patienten geeignet ist und sich weitgehend an das Bein des Patienten direkt anpassen läßt. Erfindungsgemäß geschieht dies dadurch, daß jede Lasche mit ihrer zugehörigen Schelle durch eine von außen aufgesetzte, auswechselbare Längsschiene über Einstellmittel verbunden ist, die im wesentlichen im Verbindungsbereich Lasche / Längsschiene in der Draufsicht die Winkellage zwischen der Schellenachse und der Achse der Gelerke und im Verbindungsbereich Längsschiene / Schelle den Abstand der Schelle von den Gelenken bestimmen.

Diese so gestaltete Orthese ermöglicht ihre direkte Anpassung an das Bein des Patienten in zweierlei Weise, nämlich einerseits durch Anpassung der Schellenachse in Bezug auf die Gelenke der Orthese, wodurch die erforderliche Rechts-/Linkseinstellung erfolgt, außerdem eine Anpassung an die jeweilige Längsrichtung des Ober- und Unterschenkels (O-bzw. X-Bein), andererseits eine Anpassung an die Länge des Beines durch entsprechende Einstellung der Schellen auf den jeweils erforderlichen Abstand von den Gelenken der Orthese. Dabei erforderliche therapeutische Wirkungen, z.B. das Entgegenwirken gegen O- bzw. X-Beine, wird dabei durch geeignete Einstellung ebenfalls erzielt. Die jeweilige Festlegung der Einstellmittel erfolgt durch Anlegen der Orthese an das Bein des Patienten direkt an diesem, wobei nacheinander die Einstellmittel ausgerichtet und schließlich festgelegt werden.

Die Einstellmöglichkeit der Orthese läßt sich vorteilhaft dadurch erweitern, daß zwecks Größenverstellung die Laschen in zwei jeweils einen Arm überlappende Verlängerungen auslaufen und mit dem betreffenden Arm über weitere Einstellmittel verbunden sind, die in Längsrichtung der Arme die Länge der Überlappung bestimmen. Durch die Einstellung des jeweiligen Grades der Überlappung von Verlängerung der Lasche und Arm ergeben sich folgende Anpassungsmöglichkeiten: Durch Verringerung der Überlappung, d.h. weiteres Herausrücken der Arme, werden wegen des Umfassens der Kniescheibe durch die Arme die beiden Gelenke der Orthese nach außen gerückt, so daß ein breiteres Knie in der Orthese untergebracht werden kann. Außerdem wird dabei der umfaßte Raum vergrößert, d.h. die beiden Laschen rücken voneinander weg, so daß die Kniescheibe gewissermaßen auf einem größeren Kreis umfaßt wird. Dabei ergibt sich weiterhin der Effekt, daß die Lage der durch die beiden Gelenke hindurchgehenden Achse exakt an die gedachte Bewegungsachse des Kniegelenkes angepaßt werden kann. Es ist schließlich auch der jeweilige Grad der Überlappung individuell, also unsymmetrisch einstellbar, womit jede Anpassung an axiale Verkrümmungen des Beins ermöglicht ist.

Um eine Stützung des Ober- bzw. Unterschenkels über eine besonders große Länge zu ermöglichen, werden zweckmäßig Längsschienen unterschiedlicher Längen vorgesehen, die mit mehreren Einstellmitteln für die Anordnung mehrerer Schellen versehen sind. Aufgrund des Einsatzes von Längsschienen unterschiedlicher Länge ist es möglich, die betreffende Schelle in entsprechendem Abstand vom Kniegelenk zu befestigen. Darüberhinaus ermöglicht die Längsschiene mit mehreren Einstellmitteln auch die Anordnung mehrer Schellen, womit sich eine Übereinander- bzw. Untereinander-Anordnung von Schellen ergibt, die das betreffende Bein auf einer großen Länge je nach Indikation abzustützen vermögen.

Die Schellen bildet man zweckmäßig konisch der Anatomie folgend aus. Die den Gelenken benachbarten Schellen öffnen sich dann nach außen hin und folgen damit der Verdickung des Oberschenkels bzw. der Wade in Richtung vom Kniegelenk weg.

Um ein weitgehend beschwerdeloses Tragen der Orthese zu ermöglichen, sind dieselben an ihren Enden mit Gurte aufnehmenden Schlitzen solcher Richtung versehen, daß die Gurte flächig am Bein anliegen. Auf diese weise wird vermieden, daß je nach Einstellung der Schelle die Gurte etwa nur mit einer Kante am Bein anliegen, dadurch Überdruckstellen erzeugen und durch wechselnden Muskeldruck bei der Bewegung eine unvorteilhafte Verschiebung hervorrufen.

Zweckmäßig bildet man die Gurte symmetrisch mit Klettenverschlußauflage zum gleichzeitig beidseitigen Anziehen aus. Hierdurch ergibt sich ein gleich starker Zug auf die Schlitzösen, so daß die Orthese beim Anlegen sich gegenüber dem Bein nicht verschiebt. Diese Gestaltung der Gurte ermöglicht außerdem das leichte Auswechseln, insbesondere zum Waschen, außerdem den Übergang auf längere oder kürzere Gurte je nach Bedarf. Dabei kann als Material für die Gurte sowohl elastischer Werkstoff als auch nicht dehnbares Material verwendet werden.

Um ein möglichst beschwerdeloses Tragen der Orthese zu ermöglichen, sind die Schellen mit Halteelementen für in die Schellen einsetzbare Polster unterschiedlicher Dicke versehen. Durch Wahl der Dicke der Polster kann dabei auf die Struktur des betreffenden Beines Rücksicht genommen werden. Außerdem wird hierdurch ermöglicht, daß die Polster leicht ausgewechselt werden können, was insbesondere für das Waschen wichtig ist.

Um die Orthese im Bereich der Kniekehle zu fixieren, werden an den beiden Gelenken drehbewegliche Schlitzösen für im Bereich der Kniekehle verlaufende Gurte vorgesehen. Die drehbeweglichen Schlitzösen ermöglichen eine Anpassung der jeweiligen Richtung des hindurchgezogenen Gurtes an die Beugung des Knies.

Um den Tragkomfort der Orthese zu steigern und ihre Halterung am Knie zu verbessern, werden an jedem Arm bzw. jeder Verlängerung drehbewegliche Schlitzösen für um die Kniekehle derart verlaufende Gurte vorgesehen, daß ausgehend von den Schlitzösen an den Armen bzw. Verlängerungen über die Schlitzösen an den Gelenken oberhalb und unterhalb der Kniekehle jeweils ein Gurtkreuz entsteht. Die Kniekehle bleibt dabei von den Gurten frei, jedoch ergibt sich aufgrund der Führung der Gurte ein doppeltes Gurtkreuz, bei dem sich insgesamt vier von hinten um das Knie erstreckende Gurtlängen ergeben, die mit relativ großer Oberfläche das Bein umspannen. Die Kniekehle wird auf diese Weise einer Reibung gegenüber dem Gurt nicht ausgesetzt. Auch hierbei besteht die Möglichkeit, je nach Indikation ein dehnbares oder nicht dehnbares Gurtmaterial zu verwenden.

Um einen möglichen seitlichen Druck auf das Kniegelenk durch die Gelenke der Orthese zu vermeiden, versieht man die Gelenke der Orthese zweckmäßig an ihrer Innenseite mit einem Klettenverschluß für das Anbringen von Polstern unterschiedlicher Dicke und Form. Durch diese Polster läßt sich die Orthese zusätzlich an unterschiedliche Kniebreiten anpassen, wobei insbesondere mit keilförmigen Polstern eine individuelle Anpassung an die jeweils gegebene Knieform möglich ist.

Die erfindungsgemäße Orthese eignet sich hervorragend dafür, mit ihren Einzelteilen zu einem Baukasten zusammengestellt zu werden, so daß auch extreme Größenunterschiede berücksichtigt werden können, z.B. Orthesen für Kinder bzw. für besonders große Erwachsene. Dies geschieht durch einen Baukasten, der die Zusammenstellung der Einzelteile der Orthese mit verschiedenen Größen zum Zusammenbau der vollständigen Orthese mittels der jeweiligen Einstellmittel enthält.

Ausführungsbeispiele der Erfindung sind in den Figuren dargestellt, es zeigen:
- Figur 1:: die Orthese in Vordersicht;
- Figur 2:: die gleiche Orthese in Seitensicht;
- Figur 3:: die Anordnung eines Gurtes an den an den Gelenken angebrachten Schlitzösen;
- Figur 4:: die Vordersicht einer Orthese mit jeweils mehreren Schellen auf jeder Seite des Knies und mit Einstellmitteln im Bereich der Arme und der Verlängerungen der Laschen;
- Figur 5:: die gleiche Orthese in Seitensicht;
- Figur 6:: die Rückansicht der Anordnung zweier sich zweifach kreuzender Gurte;
- Figur 7:: einen Baukasten zur Zusammenstellung der Orthese.

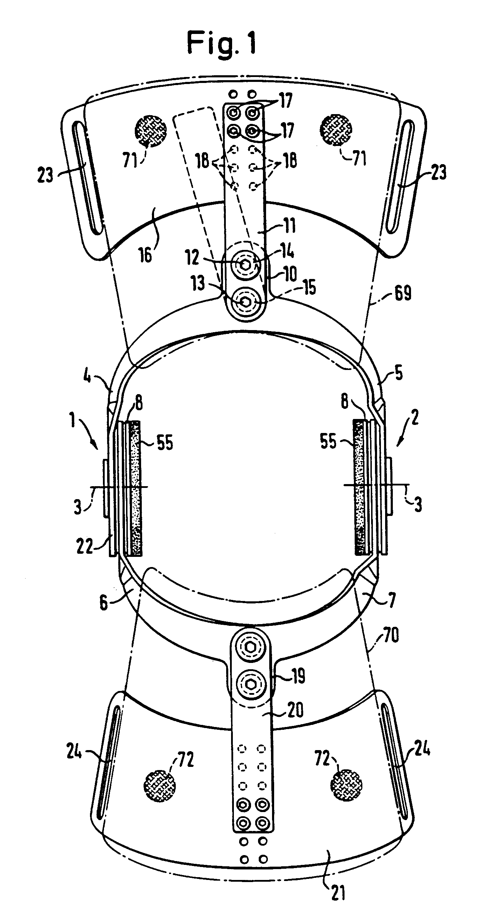

Die in der Figur 1 dargestellte Orthese besteht aus den beiden an den Seiten eines Knies angeordneten Gelenken 1 und 2, die jeweils aus der außen verschraubten Achse 3 bestehen, auf der die Arme 4 und 5 sowie 6 und 7 drehbar gelagert sind. Diese Achse 3 trägt zusätzlich innen eine Scheibe 8, die ebenfalls drehbeweglich gelagert ist, so daß sie beim Verdrehen des Gelenkes 1 bzw. 2 gegenüber dem eingeschlossenen Knie nicht mitgedreht wird und somit Reibung gegenüber dem Knie verhindert.

Die Arme 4 und 5 laufen in der Lasche 10 zusammen, die mittig in Bezug auf die gesamte Orthese angeordnet ist. Auf der Lasche 10 ist die Längsschiene 11 befestigt, und zwar mittels der beiden Schrauben 12 und 13, wobei durch die gegenüber den Schrauben 12 und 13 größeren Löcher 14 und 15 in der Lasche 10 der Längsschiene 11 eine Einstellmöglichkeit gegenüber der Lasche 10 gegeben ist. So kann beispielsweise die Längsschiene 11 in die gestrichelt gezeichnete Lage gegenüber der Lasche 10 verschwenkt werden.

An der Längsschiene 11 ist die Schelle 16 befestigt, und zwar mittels der vier Schrauben 17, die eine stabile Verbindung zwischen der Längsschiene 11 und der Schelle 16 gewährleisten. Die Schelle 16 ist mit zwei Reihen von Löchern 18 versehen, die der Anordnung der Schrauben 17 entsprechend in der Schelle 16 gebohrt sind. Die Schelle 16 kann daher gegenüber der Längsschiene 11 in verschiedene Abstandslagen in Bezug auf die Gelenke 1 und 2 an der Längsschiene 11 befestigt werden, jenachdem, welche Löcher 18 der Schelle 16 für die Befestigung ausgewählt werden.

Aufgrund dieser durch die Schrauben 12, 13 und 17 gebildeten Einstellmittel läßt sich die Schelle 16 in der oben beschriebenen Weise in zweierlei Hinsicht an das Bein des Patienten anpassen, nämlich einerseits durch Verschiebung der Längsschiene 11 gegenüber der Lasche 10 und andererseits durch Verschiebung der Schelle 16 gegenüber der Längsschiene 11.

In der gleichen Weise laufen die Arme 6 und 7 in der Lasche 19 zusammen. Die Lasche 19 ist gegenüber der Längsschiene 20 und dieser gegenüber die Schelle 21 einstellbar angeordnet und befestigt, so daß diesbezüglich auf die vorstehenden Erläuterungen im Zusammenhang mit der Schelle 16 und der Längsschiene 11 verwiesen werden kann. Im vorliegenden Falle bildet die Schelle 16 eine Oberschenkel-Schelle und die Schelle 21 eine Unterschenkel-Schelle.

Die Scheiben 8 sind nach innen hin mit einer zum Haften eines Klettenverschlusses versehenen Oberfläche versehen. Gegen diese Oberfläche sind die Polster 55 gedrückt, die auf ihrer den Scheiben 8 zugewandten Seite mit dem erwähnten Klettenverschluß versehen sind, so daß die Polster 55 an den Scheiben 8 haften. Die Polster 55 bestehen aus Kunststoff und bewirken eine weiche Abpolsterung der Gelenke 1 und 2 gegenüber dem von der Orthese eingschlossenen Knie des Patienten. Die Polster 55 stehen in unterschiedlicher Dicke und unterschiedlicher Form zur Verfügung, so daß sie je nach Bedarf in Anpassng an das Knie des betreffenden Patienten eingesetzt werden können.

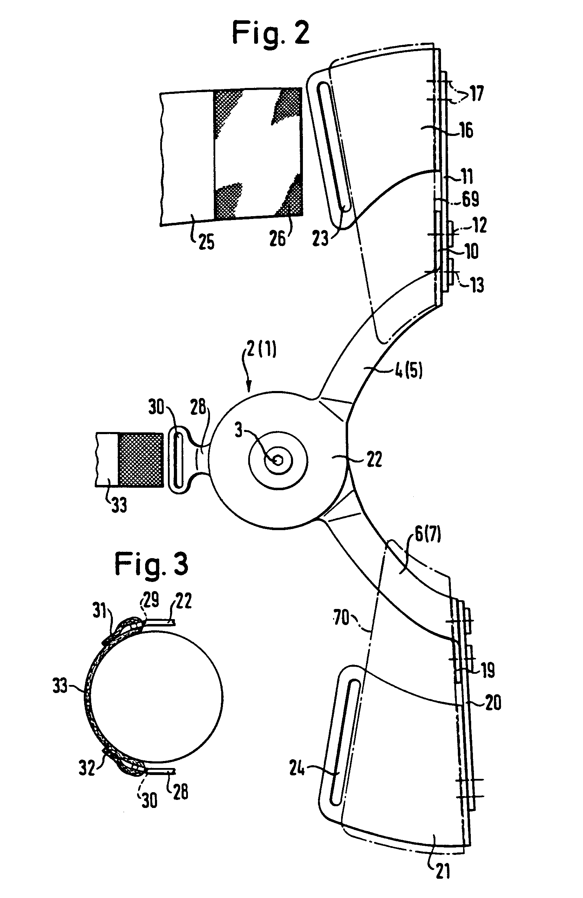

In der Figur 2 ist die gleiche Orthese in Seitenansicht dargestellt. Wie gezeigt, ist der Arm 4 einstückig mit der Platte 22 ausgebildet, die drehbar von der Achse 3 getragen wird und sich gegenüber entsprechenden Platten der Arme 5, 6 und 7 flach abstützt. Durch diese plattenförmige, an sich bekannte Ausbildung der betreffenden Teile der Gelenke 1 und 2 wird deren Verbindungssteifigkeit gewährleistet.

In den Figuren 1 und 2 sind noch jeweils an den Enden der Schellen 16 und 21 die Schlitze 23 und 24 vorgesehen, die jeweils schräg zur Längsrichtung des Oberschenkels bzw. Unterschenkels verlaufen, so daß sich durch die Schlitze hindurchgezogene Gurte flächig an das Bein anlegen können. Die Gurte sind an ihren beiden Enden jeweils mit einem Klettenverschluß versehen, so daß sie zu beiden Seiten der Schellen 16 und 21 angezogen und festgelegt werden können. In der Figur 2 ist im Zusammenhang mit der Schelle 16 der Gurt 25 dargestellt, dessen mit dem Klettenverschluß versehene Zone 26 kreuzschraffiert angedeutet ist.

Die Befestigungenanordnung des Gurtes 25 sei im Zusammenhang mit der Figur 3 erläutert. Die Figur 3 zeigt als Abschnitt die beiden Schlitzösen 27 und 28 (Schlitzöse 28 siehe Fig. 2), die drehbeweglich an den Gelenken 1 und 2 angeordnet sind. In die Schlitze 29 und 30 der Schlitzösen 27 und 28 ist jeweils das Ende 31 bzw. 32 des Gurtes 33 eingefädelt und gegenüber dem zwischen den Schlitzen 29 und 30 liegenden Teil des Gurtes 33 umgeknickt, so daß mittels Klettenverschluß die Enden 31 und 32 am Gurt 33 festhalten. Diese Art der Befestigung und Anordnung eines Gurtes ist auch bei den durch die Schlitze 23 bzw. 24 hindurchgezogenen Gurten vorgesehen (siehe Gurt 25 gemäß Fig. 2).

Aus Figur 3 ist deutlich ersichtlich, daß durch gleichseitiges und gleichmäßiges Ziehen an den Enden 31 und 32 des Gurtes 33 der Gurt 33 gleichmäßig gespannt wird, ohne daß sich dabei die Orthese gegenüber dem Bein verschiebt. Der Gurt 33 erstreckt sich bei der in Figuren 1 und 2 dargestellten Orthese über das Kniegelenk und erlaubt dadurch eine genaue Einstellung zur Übereinstimmung beider Gelenke mit dem anatomischen Kniegelenk.

Die Schellen 16 und 21 sind durch abnehmbare Polster 69 und 70 hinterlegt, die aus Schaumstoff bestehen und so das Bein des Trägers der Orthese vor Druck schützen. Die beiden Polster 69 und 70 sind in den Figuren 1 und 2 durch strichpunktierte Umrandung eingezeichnet. Die Polster erstrecken sich von den Schellen 16 und 21 her bis Ober die zugehörigen Arme 4, 5 bzw. 6, 7, so daß sich eine weitreichende Polsterung auch unter den Längsschienen 11 und 20 ergibt. Zwecks Abnehmbarkeit der Polster 69 und 70 sind die Schellen 16 und 21 an den kreuzschraffiert gezeichneten Stellen 71 und 72 mit einer Klettenverschlußoberfläche versehen, die an entsprechenden Oberflächen der Polster 69 und 70 haftet.

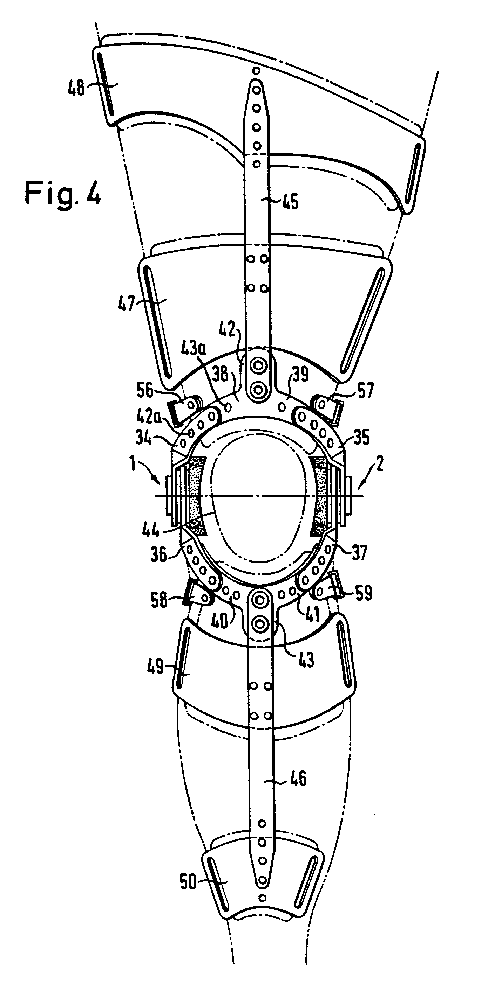

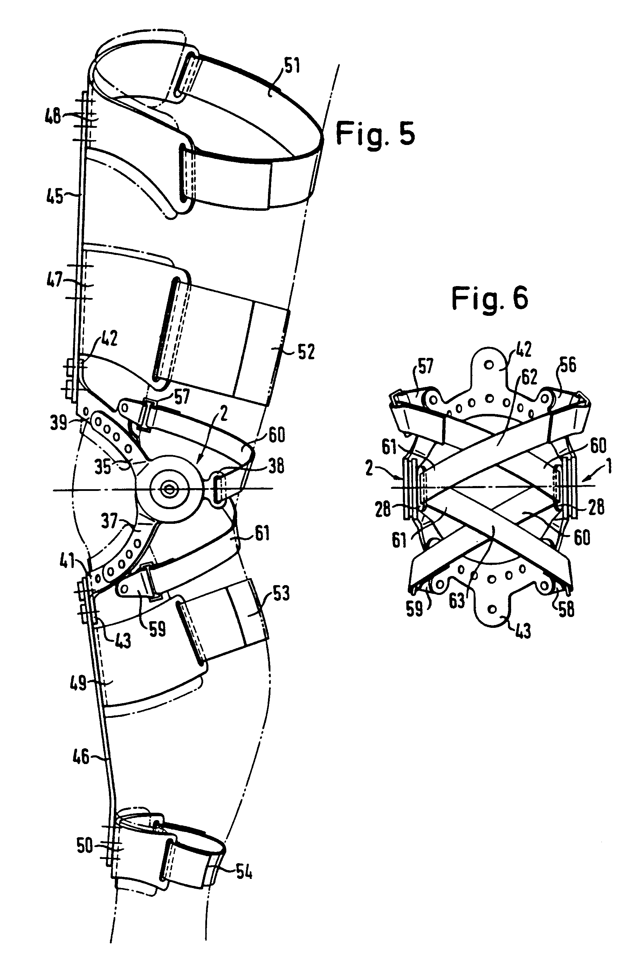

Anhand der Figuren 4 und 5 sei nunmehr eine Orthese beschrieben, die zusätzlich mit einer sich im Bereich des Kniegelenkes auswirkenden Größenverstellbarkeit versehen ist. Dabei zeigt Figur 4 die an einem Bein angebrachte Orthese in Vorderansicht und Figur 5 die gleiche Orthese in Seitenansicht.

Die Orthese enthalt wie diejenige gemäß den Figuren 1 und 2 die beiden Gelenke 1 und 2, aus denen die Arme 34 und 35 sowie 36 und 37 herausragen. Die Arme 34 und 35 bzw. 36 und 37 überlappen seitliche Verlängerungen 38, 39 bzw. 40, 41, die Bestandteile der beiden Laschen 42 und 43 sind. Die Arme 34, 35 bzw. 36, 37 sowie die Verlängerungen 38, 39 bzw. 40, 41 sind mit Löchern 42a bzw. 43a versehen, die als Einstellmittel dienen und die Fixierung der Arme zu den Verlängerungen jeweils mit unterschiedlicher Länge der Überlappung ermöglichen. In die Löcher 42, 43 werden zu diesem Zweck Schrauben eingesetzt, wodurch die gegenseitige Fixierung der Arme zu den Verlängerungen hergestellt wird. Die Löcher 42 in den Armen und die Löcher 43 in den Verlängerungen weisen jeweils den gleichen Abstand zueinander auf, so daß die jeweilige Überlappung schrittweise über einen relativ weiten Bereich eingestellt werden kann. Bei geringer Überlappung bilden die Arme 34, 35 sowie 36, 37 zusammen mit den Verlängerungen 38, 39 und 40, 41 einen relativ großen Kreis um die strichpunktiert dargestellte Kniescheibe, in dem der Abstand der Laschen 42 und 43 gegenüber der als gerade-strichpunktierte Linie gezeichneten Achse der beiden Gelenke 1 und 2 relativ groß ist. Außerdem ergibt sich bei geringer Überlappung ein entsprechend großer Abstand zwischen den beiden Gelenken 1 und 2. Umgekehrt erhält man natürlich bei großer Überlappung entsprechend umgekehrte Verhältnisse. Darüberhinaus ist es möglich, die jeweilige Überlappung individuell, insbesondere also auch unsymmetrisch einzustellen, womit, wie oben bereits erläutert ist, sich jede individuelle notwendige Anpassung an das Bein des betreffenden Patienten ermöglichen läßt.

An die Laschen 42 und 43 sind in gleicher Weise, wie zu Figuren 1 und 2 beschrieben, die Längsschienen 45 und 46 angeschraubt, die hier jeweils zwei Schellen tragen, nämlich einerseits die Schellen 47, 48 und 49, 50. Die Befestigung dieser Schellen gegenüber den Längsschienen 45 und 46 erfolgt in gleicher Weise wie zu Figuren 1 und 2 beschrieben.

Aus Figur 5 ergibt sich deutlich, daß durch die Lage der Schlitze in den Enden der Schellen 47, 48, 49 und 50 die jeweils hindurchgefädelten Gurte sich flächig an das Bein des Patienten anlegen, so daß eine Verkantung der Gurte 51, 52, 53, 54 gegenüber dem Bein nicht möglich ist.

Die in den Figuren 4 und 5 dargestellte Orthese enthält weiterhin besondere Bauteile für die Anbringung zweier Gurte im Bereich der Kniekehle. Es sind dies außer den Schlitzösen 28 (siehe auch Fig. 2) noch die Schlitzösen 56 und 57 sowie 58 und 59. Alle diese Schlitzösen sind drehbeweglich gelagert, so daß sich ihre Richtung dem Zug der an ihnen befestigten Gurte anpassen kann. Wie sich insbesondere aus Figur 6 ergibt, sind zwei Gurte 60 und 61 vorgesehen, die derart geführt sind, daß sich oberhalb und unterhalb der Kniekehle jeweils ein Gurtkreuz 62 bzw. 63 ergibt. Die Führung des Gurtes 60 verläuft dabei von der Schlitzöse 57 zu der Schlitzöse 28 am Gelenk 1 und von hier zur Schlitzöse 59. Der Gurt 61 verläuft von der Schlitzöse 56 zur Schlitzöse 28 am Lager 2 und danach zur Schlitzöse 58. Wie aus Figur 6 ersichtlich, bilden sich dabei die beiden Gurtkreuze 62 und 63 aus, zwischen denen frei die Kniekehle liegt, die infolgedessen bei Bewegung des Knies ein Scheuern durch die Gurte 60 und 61 vermeidet.

In die Figuren 4 und 5 ist entsprechend der Darstellung gemäß den Figuren 1 und 2 die Anordnung von Polstern hinter den Schellen 48, 47, 49, und 50 dargestellt, und zwar durch strichpunktierte Umrandung. Diese Polster sind in der gleichen Weise wie anhand der Figur 1 beschrieben mittels Klettenverschlüsse an den Schellen angebracht.

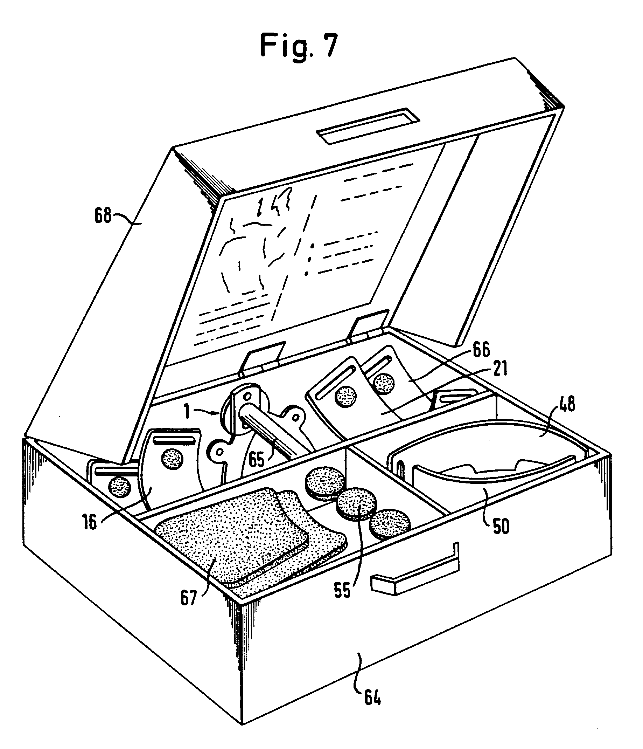

In der Figur 7 ist ein Baukasten 64 dargestellt, der die Zusammenstellung der Einzelteile der Orthese gemäß den Figuren 1 und 2 in verschiedenen Größen enthält. In dem Baukasten ist zusammengebaut diese Orthese mit ihren Schellen 16 und 21 zu sehen, bei der zwischen den Gelenken (sichtbar ist nur das Gelenk 1) das Parallelrichtgerät 65 angeordnet ist. Der Baukasten 64 enthält unter anderem die weitere Schelle 66 einer anderen Größe, außerdem Polster 67 und die an den Gelenken anbringbaren Polster 55. Weiterhin sind in dem Baukasten die aus Figur 4 und 5 ersichtlichen äußeren Schellen 48 und 50 gelagert. Auf diese Weise sind in dem Baukasten alle Einzelteile mit den jeweiligen Einstellmitteln, die auch dem Zusammenfugen dienen, untergebracht, so daß mit dem Baukasten 64 jeder Patient in Sofort-Versorgung behandelt werden kann. Auf der Innenseite des Deckels 68 des Baukastens 64 sind Zeichnungen und die Anleitung für den Zusammenbau der Orthese angebracht.

## Patentansprüche

1. Orthese für das menschliche Knie mit zwei an den Seiten des Knies angeordneten Gelenken, aus denen die Kniescheibe umfassende Arme herausragen, die paarweise über und unter der Kniescheibe jeweils in einer mittig angeordneten Lasche zusammenlaufen, von der oberhalb und unterhalb des Knies mindestens je eine Oberschenkel- und Unterscherkel-Schelle getragen werden, **dadurch gekennzeichnet**, daß jede Lasche (10, 19; 42, 43) mit ihrer zugehörigen Schelle (16, 21; 47, 48, 49, 50) durch eine von außen aufgesetzte, auswechselbare Längsschiene über Einstellmittel (12, 13, 17) verbunden ist, die im wesentlichen im Verbindungsbereich Lasche (10, 19; 42, 43) / Längsschiene (11, 20, 45, 46) in der Draufsicht die Winkellage zwischen der Schellenachse und der Achse der Gelenke (1, 2) und im Verbindungsbereich Längsschiene (11, 20, 45, 46) / Schelle (16, 21; 47, 48, 49, 50) den Abstand der Schelle von den Gelenken (1, 2) bestimmen.

2. Orthese nach Anspruch 1, **dadurch gekennzeichnet**, daß zwecks Größenverstellung die Laschen (42, 43) in zwei jeweils einen Arm (34, 35; 36, 37) überlappende Verlängerungen (38, 39; 40, 41) auslaufen und mit dem betreffenden Arm (34, 35; 36, 37) über weitere Einstellmittel (42a, 43a) verbunden sind, die in Längsrichtung der Arme (34, 35; 36, 37) die Länge der Überlappung bestimmen.

3. Orthese nach Anspruch 1 oder 2, **dadurch gekennzeichnet**, daß die Längsschienen (45, 46) mit unterschiedlicher Länge ausgebildet und mit mehreren Einstellmitteln für die Anordnung mehrerer Schellen versehen ist.

4. Orthese nach einem der Ansprüche 1 bis 3, **dadurch gekennzeichnet**, daß die den Gelenken (1, 2) benachbarten Schellen (16, 21; 47, 49) konisch der Anatomie folgend ausgebildet sind.

5. Orthese nach Anspruch 4**, dadurch gekennzeichnet**, daß die Schellen (16, 21) an ihren Enden mit Gurte (25, 33) aufnehmenden Schlitzen (23, 24) solcher Richtung versehen sind, daß die Gurte (25, 33) flächig am Bein anliegen.

6. Orthese nach einem der Ansprüche 1 bis 5, **dadurch gekennzeichnet**, daß die Gurte (25, 33) symmetrisch mit Klettenverschlußauflage (26) zum gleichzeitig beidseitigen Spannen ausgebildet sind.

7. Orthese nach einem der Ansprüche 1 bis 6, **dadurch gekennzeichnet**, daß die Schellen (16, 21) mit Halteelementen (71, 72) für in die Schellen (16, 21) einsetzbare Polster (69, 70) unterschiedlicher Dicke versehen sind.

8. Orthese nach einem der Ansprüche 1 bis 7, **dadurch gekennzeichnet**, daß an den beiden Gelenken (1, 2) drehbewegliche Schlitzösen (30) für im Bereich der Kniekehle verlaufende Gurte (33; 60, 61) vorgesehen sind.

9. Orthese nach Anspruch 8, **dadurch gekennzeichnet**, daß an jedem Arm (34, 35; 36, 37) bzw. jeder Verlängerung (38, 39; 40, 41) drehbewegliche Schlitzösen (56, 57, 58, 59) für um die Kniekehle derart verlaufende Gurte (60, 61) vorgesehen sind, daß ausgehend von den Schlitzösen (56, 57, 58, 59) an den Armen (34, 35; 36, 37) bzw. Verlängerungen (38, 39; 40, 41) über die Schlitzösen (56, 57, 58, 59) an den Gelenken (1. 2) oberhalb und unterhalb der Kniekehle jeweils ein Gurtkreuz (62, 63) entsteht.

10. Orthese nach einem der Ansprüche 1 bis 9, **dadurch gekennzeichnet**, daß die Gelenke (1, 2) an ihrer Innenseite mit einem Klettverschluß für das Anbringen von Polstern (55) unterschiedlicher Dicke und Form versehen sind.

11. Orthese nach einem der Ansprüche 1 bis 10, **gekennzeichnet durch** die Zusammenstellung ihrer Einzelteile in verschiedenen Größen in einem Baukasten zum Zusammenbau mittels der jeweiligen Einstellmittel.

## Claims

1. Orthesis for the human knee with two joints arranged at the sides of the knee and from which arms project which surround the kneecap and run in pairs above and below the kneecap to meet in each case in a centrally arranged tab which bears at least one thigh clamp and one lower leg clamp above and below the knee respectively, characterised in that each tab (10, 19; 42, 43) is connected to the appertaining clamp (16, 21; 47, 48, 49, 50) by an externally mounted replaceable longitudinal rail via adjusting means (12, 13, 17) which determine the angular position in plan view between the clamp axis and the axis of the joints (1, 2) essentially in the region of the connection between the tab (10, 19; 42. 43) and the longitudinal rail (11, 20, 45, 46) and determine the distance of the clamp from the joints (1, 2) in the region of the connection between the longitudinal rail (11, 20, 45, 46) and the clamp (16, 21; 47, 48, 49, 50).

2. Orthesis as claimed in claim 1, characterised in that for the purpose of size adjustment the tabs (42, 43) each terminate in extensions (38, 39; 40, 41) which each overlap one arm (34, 35; 36, 37), the said tabs being connected to the relevant arm (34, 35; 36, 37) by further adjusting means (42a, 43a) which determine the length of the overlap in the longitudinal direction of the arms (34, 35: 36, 37).

3. Orthesis as claimed in claims 1 or 2, characterised in that the longitudinal rails (45, 46) are constructed with differing lengths and are provided with several adjusting means for the arrangement of several clamps.

4. Orthesis as claimed in one of claims 1 to 3, characterised in that the clamps (16, 21; 47, 49) adjacent to the joints (1, 2) are constructed conically following the anatomy.

5. Orthesis as claimed in claim 4, characterised in that the clamps (16, 21) are provided at their ends with slots (23, 24) to receive straps (25, 33). the slots being aligned in such a direction that the straps (25, 33) lie flat on the leg.

6. Orthesis as claimed in one of claims 1 to 5, characterised in that the straps (25, 33) are constructed symmetrically with a burred fabric fastening (26) for simultaneous gripping on both sides.

7. Orthesis as claimed in one of claims 1 to 6, characterised in that the clamps (16, 21) are provided with retaining elements (71, 72) for pads (69, 70) of differing thickness which can be set into the clamps (16, 21).

8. Orthesis as claimed in one of claims 1 to 7, characterised in that rotatable slotted eyelets (30) for straps (33; 60, 61) running in the region of the hollow of the knee are provided on both joints (1, 2).

9. Orthesis as claimed in claim 8, characterized in that rotatable slotted eyelets (56, 57. 58, 59) are provided on each arm (34, 35; 36, 37) or each extension (38, 39: 40, 41) for straps (60, 61) running around the hollow of the knee in such a way that a strap cross (62, 62) is produced in each case above and below the hollow of the knee, starting from the slotted eyelets (56, 57, 58, 50) on the arms (34, 35; 36, 37) or extensions (38, 39: 40, 41) via the slotted eyelets (56, 57, 58, 59) on the joints (1, 2).

10. Orthesis as claimed in one of claims 1 to 9, characterized in that the joints (1, 2) are provided on their inner face with a burred fabric fastening for the attachment of pads (55) of differing thickness and shape.

11. Orthesis as claimed in one of claims 1 to 10, characterised by the arrangement of its individual parts in different sizes in a case for assembly by means of the appropriate adjusting means.

## Revendications

1. Orthèse pour le genou humain, comprenant des articulations disposées des deux côtés du genou, depuis lesquelles se projettent des bras entourant la rotule, convergeant par paires respectivement au-dessus et en dessous de la rotule, au niveau d'une attache disposée de façon médiane, qui porte, audessus et en dessous du genou respectivement, au moins un étrier de partie supérieure et un étrier de partie inférieure, caractérisée en ce que chaque attache (10, 19; 42, 43) est reliée à son étrier associé (16, 21; 47, 48, 49, 50) par l'intermédiaire d'un longeron interchangeable, rapporté extérieurement, et par l'intermédiaire de moyens (12, 13, 17) de réglage qui, au niveau essentiellement de la liaison attache (10, 19; 42, 43)/ longeron (11, 20, 45, 46), déterminent la position angulaire, vue du dessus, entre l'axe de l'étrier et l'axe des articulations (1, 2) et, au niveau de la liaison longeron (11, 20, 45, 46)/ étrier 16, 21; 47, 48, 49, 50), déterminent la distance entre l'étrier et les articulations (1, 2).

2. Prothèse orthopédique selon la revendication 1, caractérisée en ce qu'afin de permettre un réglage de la taille, les attaches (42, 43) se terminent dans deux prolongements (38, 39; 40, 41) recouvrant respectivement un bras (34, 35; 36, 37) et reliés au bras concerné (34, 35; 36, 37) par l'intermédiaire d'autres moyens (42a, 43a) de réglage, qui déterminent la longueur de recouvrement dans le sens longitudinal des bras (34, 35; 36, 37).

3. Prothèse orthopédique selon la revendication 1 ou 2, caractérisée en ce que les longerons (45, 46) sont réalisés avec des longueurs différentes et sont pourvus de plusieurs moyens de réglage permettant l'installation de plusieurs étriers.

4. Prothèse orthopédique selon l'une des revendications 1 à 3, caractérisée en ce que les étriers (16, 21; 47, 49) contigus aux articulations (1, 2) sont réalisés de façon conique, suivant l'anatomie.

5. Prothèse orthopédique selon la revendication 4, caractérisée en ce que les étriers (16, 21) sont pourvus, à leurs extrémités, de fentes (23, 24) recevant des ceintures (25, 33) , les fentes étant orientées de façon telle que les ceintures (25, 33) s'appliquent à plat sur la jambe.

6. Prothèse orthopédique selon l'une des revendications 1 à 5, caractérisée en ce que les ceintures (25, 33) sont réalisées de façon symétrique, comprenant une face d'appui pour attaches à agrafage (26), permettant la tension simultanée de celles-ci des deux côtés.

7. Prothèse orthopédique selon l'une des revendications 1 à 6, caractérisée en ce que les étriers (16, 21) sont pourvus d'éléments (71, 72) de fixation de coussins (69, 70) de différentes épaisseurs, pouvant être insérés dans les étriers (16, 21).

8. Prothèse orthopédique selon l'une des revendications 1 à 7, caractérisée en ce que des oeillets à fente (30) pivotants, destinés aux ceintures (33; 60, 61) s'étendant au niveau du jarret, sont prévus sur les deux articulations (1, 2).

9. Prothèse orthopédique selon la revendication 8, caractérisée en ce que des oeillets à fente pivotants (56, 57, 58, 59) sont prévus sur chaque bras (34, 35; 36, 37), voire sur chaque prolongement (38, 39; 40, 41), ces oeillets étant destinés aux ceintures (60, 61) s'étendant de telle façon autour du jarret, qu'elles créent, à partir des oeillets à fente (56, 57, 58, 59) situés sur les bras (34, 35; 36, 37), voire sur les prolongements (38, 39; 40, 41), et en passant par les oeillets à fente (56, 57, 58, 59) situés sur les articulations (1, 2), un croisement (62, 63) des ceintures respectivement au-dessus et en dessous du jarret.

10. Prothèse orthopédique selon l'une des revendications 1 à 9, caractérisée en ce que les articulations (1, 2) sont pourvues, à leur face intérieure, d'une attache à agrafage permettant la mise en place de coussins (55) de différentes épaisseurs et de différentes formes.

11. Prothèse orthopédique selon l'une des revendications 1 à 10, caractérisée par le rassemblement de ses pièces détachées, en différentes tailles, en un jeu d'éléments modulaires permettant l'assemblage de celle-ci par l'intermédiaire des moyens de réglage correspondants.
